(19) Europäisches Patentamt
European Patent Office
Office européen des brevets

(11) EP 2 343 372 B1

(12) EUROPEAN PATENT SPECIFICATION

(45) Date of publication and mention of the grant of the patent:
26.11.2014 Bulletin 2014/48

(51) Int Cl.:
C12Q 1/68 (2006.01)   C12N 15/09 (2006.01)
C12M 1/00 (2006.01)   C12M 1/34 (2006.01)
A61K 39/395 (2006.01)   A61P 29/00 (2006.01)

(21) Application number: 09817872.6

(22) Date of filing: 01.10.2009

(86) International application number:
PCT/JP2009/067187

(87) International publication number:
WO 2010/038840 (08.04.2010 Gazette 2010/14)

(54) **METHOD FOR PREDICTING PHARMACOLOGICAL EFFICACY OF ANTI-TNF ANTIBODY PREPARATION ON RHEUMATOID ARTHRITIS, AND PHARMACOLOGICAL EFFICACY PREDICTION APPARATUS**

VERFAHREN ZUR VORHERSAGE DER PHARMAKOLOGISCHEN WIRKSAMKEIT EINER ANTI-TNF-ANTIKÖRPERZUBEREITUNG ZUR BEHANDLUNG VON RHEUMATOIDER ARTHRITIS SOWIE VORRICHTUNG ZUR VORHERSAGE DER PHARMAKOLOGISCHEN WIRKSAMKEIT

PROCÉDÉ DE PRÉDICTION DE L'EFFICACITÉ PHARMACOLOGIQUE D'UNE PRÉPARATION D'ANTICORPS ANTI-TNF SUR LA POLYARTHRITE RHUMATOÏDE, ET APPAREIL DE PRÉDICTION DE L'EFFICACITÉ PHARMACOLOGIQUE

(84) Designated Contracting States:
AT BE BG CH CY CZ DE DK EE ES FI FR GB GR HR HU IE IS IT LI LT LU LV MC MK MT NL NO PL PT RO SE SI SK SM TR

(30) Priority: 03.10.2008 JP 2008258235

(43) Date of publication of application:
13.07.2011 Bulletin 2011/28

(73) Proprietor: Kaytee Bio Co. & Ltd.
Tokyo 104-0061 (JP)

(72) Inventors:
• TAKEUCHI, Tsutomu
  Tokyo 160-8582 (JP)
• TSUZAKA, Kensei
  Chuo-ku,
  Tokyo 104-0061 (JP)

(74) Representative: Hill, Christopher Michael
Page White & Farrer
Bedford House
John Street
London, WC1N 2BF (GB)

(56) References cited:
WO-A1-2006/092530

• LEQUERRE T ET AL: "Autoantibodies, metalloproteinases and bone markers in rheumatoid arthritis patients are unable to predict their responses to infliximab", RHEUMATOLOGY, OXFORD UNIVERSITY PRESS, LONDON, GB, vol. 46, no. 3, 1 March 2007 (2007-03-01), pages 446-453, XP002486683, ISSN: 1462-0324, DOI: 10.1093/RHEUMATOLOGY/KEL262 [retrieved on 2006-08-09]
• C TROCME ET AL: "Apolipoprotein A-I and platelet factor 4 are biomarkers for infliximab response in rheumatoid arthritis", ANNALS OF THE RHEUMATIC DISEASES, vol. 68, no. 8, 29 July 2008 (2008-07-29), pages 1328-1333, XP55021751, ISSN: 0003-4967, DOI: 10.1136/ard.2008.093153
• AMANDA J. FOSANG ET AL: "ADAMTS-5: THE STORY SO FAR", EUROPEAN CELLS AND MATERIALS, vol. 15, 5 February 2008 (2008-02-05), pages 11-26, XP55021758, ISSN: 1473-2262
• LEQUERRE T. ET AL.: 'Gene profiling in white blood cells predicts infliximab responsiveness in rheumatoid arthritis' ARTHRITIS RES. THER. vol. 8, no. 4, 2006, page R105, XP021020585

- SEGUIN C.A. ET AL.: 'Differential regulation of matrix degrading enzymes in a TNFalpha-induced model of nucleus pulposus tissue degeneration' MATRIX BIOL. vol. 25, no. 7, September 2006, pages 409 - 418, XP025079554
- 'Dai 53 Kai Nippon Rheumatism Gakkai Sokai.Gakujutsu Shukai (Dai 18 Kai Kokusai Rheumatism Symposium) Program. Shorokushu', 19 March 2009 article KENSEI TSUZAKA ET AL.: 'Toyo Zen Kecchu ADAMTS5 Hatsugenryo o Moto ni shita RA ni Taisuru Infliximab Yukosei Yosoku', page 274, XP008147968

**Description**

Technical Field

The present invention relates to a method and an apparatus for predicting a pharmaceutical efficacy of an anti-TNFα antibody drug against rheumatoid arthritis by employing as an index an amount of ADAMTS5 contained in a sample derived from a subject.

Background Art

In recent years, biologies targeting to TNFα (tumor necrosis factor-α) have been used for the purpose of remitting rheumatoid arthritis, and their efficacy has been recognized. Infliximab (INF) is a biologic containing an anti-TNFα monoclonal antibody and targeting to TNFα. It has already been found that infliximab suppresses activity of rheumatoid arthritis from a clinical index referred to as DAS (Disease activity score) 28. Infliximab not only suppresses activity of rheumatoid arthritis, but also suppresses bone destruction associated with rheumatoid arthritis.

Regardless of such remarkable efficacy, some cases with rheumatoid arthritis do not respond to infliximab. Furthermore, infliximab is prone to cause, as adverse side effects, infections such as Pneumocystis pneumonia and pulmonary tuberculosis. Thus, predicting infliximab efficacy prior to administration thereof allows us to administer infliximab to cases on which it assuredly takes effects, preventing the occurrence of unnecessary adverse side effects.

Some attempts to identify an INF efficacy predictive factor have already been reported (see, for example, NPLs 1 and 2). Many of them analyzed expressions of genes associated with infliximab responders by an algorithm through a retrospective study comparing infliximab responders with infliximab nonresponders. No infliximab efficacy predictive factor with high reliability has been identified that could be confirmed in a prospective study, etc.

It is known that ADAMTS (a disintegrin and metalloproteinase with thrombospondin motifs) 5 is an aggrecanase that belongs to the ADAMTS family and involved in cartilage destruction. In particular, ADAMTS5 deficiency results in preventing cartilage destruction in a murine model of degenerative arthritis.

However, it has not been known whether or not the expression level of ADAMTS5 associates with an efficacy of an anti-TNFα antibody drug against rheumatoid arthritis.

In PL 1, an *in vitro* method for determining, or monitoring, the response of a rheumatoid arthritis patient to a treatment against a cytokine involved in the inflammatory process of the disease is disclosed. The expression of the gene encoding synoviolin is measured.

However, in NPL 3 Lequerre and co-workers rule out that metalloproteases and bone markers may be used as markers for predicting patient responses to treatment.

Citation List

Non-Patent Literature

NPL 1: Laquerre T, Gauthier-Jauneau A, Bansard C, Derambure C, Hiron M, Vittecoq1 O, Daveau M, Mejjad O, Daragon A, Tron F, Le Loet X, Salier J-P. Arthritis Res Ther 2006; 8: R105
NPL 2: Trocme C, Marotte H, Baillet A, Pallot-Prades B, Garin J, Grange L, Miossec P, Tebib J, Berger F, Nissen MJ, Juvin R, Morel F, Gaudin P. Ann Rheum Dis 2008. Epub ahead of print
NPL 3: Laquerre T. et al. "Autoantibodies, metalloproteinases and bone markers in rheumatoid arthritis patients are unable to predict their responses to infliximab" Rheumatology, 2007; 46:446-453

Patent Literature

PL 1: WO 2006/092530

Summary of Invention

Technical Problem

The present invention aims to solve the above existing problems and achieve the following objects. Specifically, an object of the present invention is to provide a method and an apparatus for predicting a pharmaceutical efficacy of an anti-TNFα antibody drug against rheumatoid arthritis, which can predict an efficacy of an anti-TNFα antibody drug against rheumatoid arthritis with high reliability and in a simple manner as well as can predict activity of rheumatoid arthritis after administration of the anti-TNFα antibody drug, resulting in that the anti-TNFα antibody drug can be administered to cases on which it assuredly takes effects to prevent the occurrence of unnecessary adverse side effects.

Solution to Problem

The present inventors conducted extensive studies to solve the above problems and have found that the lower the amount of ADAMTS5 contained in peripheral blood of a patient with rheumatoid arthritis before administration of infliximab, the more efficient infliximab against rheumatoid arthritis. No prospective study has showed that the efficacy of infliximab could be predicted by determining the amount of ADAMTS5 contained in blood before administration of infliximab. Thus, this finding is first obtained by the present inventors.

The term "retrospective study" refers to a study that analyzes past data and present data, while the term "prospective study" refers to a study that follows up a phenomenon occurring in the future. The obtained results are more reliable in the prospective study than in the retrospective study. This is because the retrospective study is performed on the already known matter and thus easily involves due to researchers' bias while the prospective study is performed on unknown matter and thus is free from researchers' bias. Therefore, the prospective study is superior to the retrospective study.

The present invention is based on the above finding obtained by the present inventors. Means for solving the above existing problems are as follows.

<1> A method for predicting a pharmaceutical efficacy of an anti-TNFα antibody drug against rheumatoid arthritis, the method comprising:

> measuring an amount of ADAMTS5 contained in a sample derived from a subject, and
> determining that the anti-TNFα antibody drug is efficient against rheumatoid arthritis if the amount of ADAMTS5 is less than a prescribed value,
> wherein the amount of ADAMTS5 contained in the sample derived from the subject is an expression level of ADAMTS5 mRNA, and
> wherein the prescribed value is 0.6 x 1/25 as the expression level of ADAMTS5 mRNA measured using β-actin as an endogenous control (ADAMTS5/β-actin).

<2> The method according to <1>, wherein the anti-TNFα antibody drug is Infliximab (INF).
<3> The method according to <1> or <2>, wherein the expression level of ADAMTS5 mRNA is measured by a real-time PCR method.
<4> An apparatus for predicting a pharmaceutical efficacy of an anti-TNFα antibody drug against rheumatoid arthritis, the apparatus comprising:

> a unit configured to measure an amount of ADAMTS5 contained in a sample derived from a subject, by measuring an expression level of ADAMTS5 mRNA and
> an electronic calculator configured to determine that the anti-TNFα antibody drug is efficient against rheumatoid arthritis if the amount of ADAMTS5 is less than a prescribed value,
> wherein the prescribed value is 0.6 x 1/25 as the expression level of ADAMTS5 mRNA measured using β-actin as an endogenous control (ADAMTS5/β-actin).

<5> The apparatus according to <4>, wherein the anti-TNFα antibody drug is Infliximab (INF).
<6> The apparatus according to <4> or <5>, wherein the expression level of ADAMTS5 mRNA is measured by a real-time PCR method.

Advantageous Effects of Invention

The present invention can solve the above existing problems to achieve the following objects. The present invention can provide a method and an apparatus for predicting a pharmaceutical efficacy of an anti-TNFα antibody drug against rheumatoid arthritis, which can predict the efficacy of the anti-TNFα antibody drug against rheumatoid arthritis with high reliability and in a simple manner as well as can predict activity of rheumatoid arthritis after administration of the anti-TNFα antibody drug, resulting in that the anti-TNFα antibody drug can be administered to cases on which it assuredly takes effects to prevent the occurrence of unnecessary adverse side effects.

Brief Description of Drawings

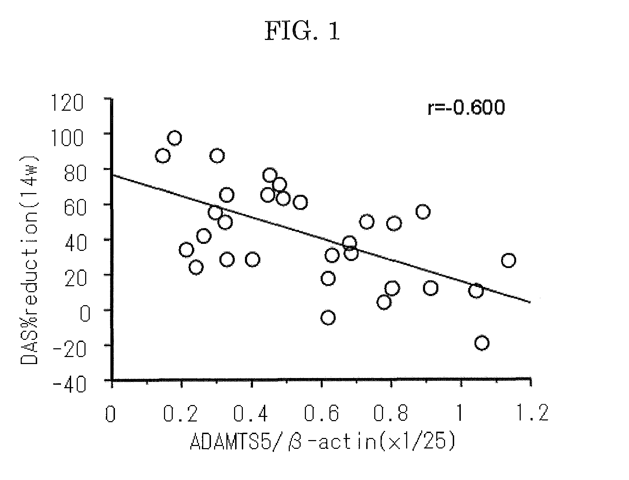

Fig. 1 is a graph indicating correlation between the expression level of ADAMTS5 mRNA before administration of infliximab and the change of DAS28 at the end of 14 weeks of infliximab administration (DAS% reduction).

Description of Embodiments

(Method and apparatus for predicting a pharmaceutical efficacy of an anti-TNFα antibody drug against rheumatoid arthritis)

A method of the present invention for predicting a pharmaceutical efficacy of an anti-TNFα antibody drug against rheumatoid arthritis includes at least measuring an amount of ADAMTS5 contained in a sample derived from a subject (measuring step) and determining whether or not the anti-TNFα antibody drug is efficient against rheumatoid arthritis by employing the amount of ADAMTS5 as an index (determining step); and, if necessary, further includes other steps.

An apparatus of the present invention for predicting a pharmaceutical efficacy of an anti-TNFα antibody drug against rheumatoid arthritis includes at least a unit configured to measure an amount of ADAMTS5 contained in a sample derived from a subject (measuring unit) and a unit configured to determine whether or not the anti-TNFα antibody drug is efficient against rheumatoid arthritis by employing the amount of ADAMTS5 as an index (determining unit); and, if necessary, further includes other units.

<Measuring step and measuring unit>

The measuring step is a step of measuring an amount of ADAMTS5 contained in a sample derived from a subject.

The measuring unit is a unit configured to measure an amount of ADAMTS5 contained in a sample derived from a subject.

-Sample derived from subject-

The sample derived from a subject is not particularly limited and may be appropriately selected depending on the intended purpose. Examples thereof include peripheral blood, a synovial membrane and synovial fluid. In particular, peripheral blood is preferred because it is easy to collect.

The subject is not particularly limited and may be appropriately selected depending on the intended purpose. Examples thereof include human.

-ADAMTS5-

The ADAMTS (a disintegrin and metalloproteinase with thrombospondin motifs) 5 is an aggrecanase that belongs to the ADAMTS family and involved in cartilage destruction.

The nucleotide sequence of ADAMTS5 gene is known in human, for example. The nucleotide sequence is easily available from public databases such as GenBank (NCBI). For example, the nucleotide sequence of human ADAMTS5 gene is available under NCBI accession number NM_007038.

-Measurement of amount-

The method for measuring the amount of ADAMTS5 contained in the sample derived from a subject is a method for measuring an mRNA expression level.

The method for measuring the mRNA expression level is not particularly limited and may be appropriately selected depending on the intended purpose. Examples thereof include a polymerase chain reaction (PCR) method, a real-time PCR method, a DNA array method, and a Northern blotting method.

The apparatus for measuring the mRNA expression level is not particularly limited and may be appropriately selected depending on the intended purpose. Examples thereof include a PCR apparatus, a real-time PCR apparatus, a DNA array apparatus, and a Northern blotting apparatus. Each of the above apparatuses can be suitably used as the measuring unit.

The real-time PCR method is not particularly limited and may be appropriately selected depending on the intended purpose. Examples thereof include a method that includes constructing a standard curve and quantifying a sample using the standard curve (standard curve method).

The template DNA control used for the standard curve method is not particularly limited and may be appropriately selected depending on the intended purpose. Examples thereof include the whole cDNA obtained and purified from healthy human, and a plasmid into which ADAMTS5 cDNA has been incorporated.

The primers used in the real-time PCR method are not particularly limited, so long as ADAMTS5 can be amplified, and may be appropriately selected depending on the intended purpose.

The endogenous control used in the real-time PCR method is not particularly limited and may be appropriately selected depending on the intended purpose. Examples thereof include β-actin and GAPDH (glyceraldehyde-3-phosphate dehydrogenase).

<Determining step and determining unit>

The determining step is a step of determining whether or not the anti-TNFα antibody drug is efficient against rheumatoid arthritis by employing the amount of ADAMTS5 as an index.

The determining unit is a unit configured to determine whether or not the anti-TNFα antibody drug is efficient against rheumatoid arthritis by employing the amount of ADAMTS5 as an index.

-Anti-TNFα antibody drug-

The anti-TNFα (tumor necrosis factor-α) antibody drug is not particularly limited and may be appropriately selected depending on the intended purpose. For example, infliximab (INF) is preferred.

-Index-

As the index, an amount of ADAMTS5 contained is used.

-Determination-

The method determines whether or not the anti-TNFα antibody drug is efficient against rheumatoid arthritis if the expression level of ADAMTS5 mRNA (ADAMTS5/β-actin) is less than $0.6 \times 1/25$ when using the amount of ADAMTS5 as the index (details will be described in the below Example 1).

The determining unit is an electronic calculator (a computer). The above unit can be suitably used as the determining unit.

The determination enables DAS28 to be predicted after administration of an anti-TNFα antibody drug.

The term "DAS28" is an abbreviation of Disease Activity Score which is established by the European League Against Rheumatism (EULAR) in order to score activity of rheumatoid arthritis and described in Fransen J, et al. Clin Exp Rheumatol 2005; 23 (Suppl. 39): S93-S99.

The method for predicting DAS28 after administration of an anti-TNFα antibody drug is not particularly limited and may be appropriately selected depending on the intended purpose. One exemplary method for predicting DAS28 after administration of an anti-TNFα antibody drug includes determining an amount of ADAMTS5 mRNA contained in peripheral blood of a patient with rheumatoid arthritis, applying the amount of ADAMTS5 mRNA to the graph referred to in the below Example 1 (see Fig. 1) and calculating the percentage of DAS28 reduced from the score before administration of the anti-TNFα antibody drug.

Examples

The present invention will next be described in detail by way of Examples, which should not be construed as limiting the present invention thereto.

(Example 1)

<Efficacy prediction of infliximab against rheumatoid arthritis by employing an amount of ADAMTS5 as an index>

Thirty patients with rheumatoid arthritis were enrolled in this study. They visited the Division of Rheumatology, Department of Internal Medicine, Saitama Medical Center, Saitama Medical University from October 2007 to April 2008 and treated with infliximab.

-Measuring step-

Measurement of the expression level of ADAMTS5 mRNA in peripheral blood

Peripheral blood (2.5 mL) was collected from the patients with rheumatoid arthritis before administration of infliximab, and then placed in a PAXgene Blood RNA Tube (registered trademark, product of Becton, Dickinson and Company Japan), followed by isolating total RNA using a PAXgene Blood RNA Kit (registered trademark, product of PreAnalytiX GmbH). The total RNA was converted to whole cDNA using a reverse transcriptase. Using the whole cDNA as a template DNA, the expression level of ADAMTS5 mRNA was measured by a real-time PCR method using Taqman (registered trademark) Gene Expression Assay (product of Applied Biosystems). The primers and probes used were a primer-probe set (TaqMan Gene (registered trademark) Expression Assay 00199841_ml, product of Applied Biosystems).
The amount of ADAMTS5 mRNA obtained above was quantified as a relative value to the level of β-actin (endogenous control) mRNA.
The primers and probes of β-actin were a primer-probe set (Pre-Developed TaqMan (registered trademark) Assay Reagents (Human ACTB, NM_001101, product of Applied Biosystems)).

-Determining step-

The activity of the patient with rheumatoid arthritis was determined according to DAS28 before administration of infliximab and at 14 weeks after infliximab administration (DAS28 (0w) and DAS28 (14w)).
According to the EULAR response criteria using DAS28 (14w), the patients were determined as "good response," "moderate response" or "no response".
The EULAR response criteria are established by European League Against Rheumatism (EULAR) and described in Fransen J, et al. Clin Exp Rheumatol 2005; 23 (Suppl. 39): S93-S99.
Regarding to infliximab efficacy at 14 weeks after administration of infliximab, good response cases, moderate response cases and nonresponse cases were respectively 16, 6, and 8 according to the EULAR response criteria. Additionally, 14 cases entered remission (DAS28 (14w) < 2.6).

--Comparison of R group with NR group--

The 30 cases with rheumatoid arthritis, who had been treated with infliximab, were categorized into the R group (moderate response + good response) and the NR group (no response). The expression level of ADAMTS5 mRNA before administration of infliximab was compared between the R group and the NR group by a real-time PCR method with a standard curve method using the whole cDNA from healthy human as a template DNA control. As a result, the ADAMTS5 mRNA expression level in the R group was significantly lower than that in the NR group (see Table 1).

Table 1

| | ADAMTS5/ $\beta$ -actin(x1/25) | |
|---|---|---|
| R group ( n = 22 ) | 0.467 ± 0.254 | |
| NR group ( n = 8 ) | 0.815 ± 0.031 | p = 0.0014 |

Also, as shown in Table 2, the cases whose ADAMTS5 mRNA expression level was lower than $0.6 \times 1/25$ were classified into the low ADAMTS5 group (Low), while the cases whose ADAMTS5 mRNA expression level was equal to or higher than $0.6 \times 1/25$ were classified into the high ADAMTS5 group (High).

Table 2

| ADAMTS5 | R | NR | Total |
|---|---|---|---|
| Low | 16 | 0 | 16 |
| High | 6 | 8 | 14 |

(continued)

| ADAMTS5 | R | NR | Total |
|---|---|---|---|
| Total | 22 | 8 | 30 |
| $\chi^2$ p = 0.0004 | | | |

Based on the classification in Table 2, percentages of sensitivity, specificity, positive predictive value, and negative predictive value were calculated according to the following equations. The results are shown in Table 3.

$$\text{Sensitivity (\%)} = A/(A + C) \times 100$$

$$\text{Specificity (\%)} = D/(D + B) \times 100$$

$$\text{Positive predictive value (PPV) (\%)} = A/(B + A) \times 100$$

$$\text{Negative predictive value (NPV) (\%)} = D/(D + C) \times 100$$

where A to D means as follows:

A: Number of cases classified into the low ADAMTS5 group and the R group
B: Number of cases classified into the low ADAMTS5 group and the NR group
C: Number of cases classified into the high ADAMTS5 group and the R group
D: Number of cases classified into the highADAMTS5 group and the NR group

Table 3

| Sensitivity | 72.7% |
|---|---|
| Specificity | 100% |
| PPV | 100% |
| NPV | 57.1% |

As is clear from Table 3, the positive predictive value (PPV), which predicts that the cases classified into the low ADAMTS5 group would be classified into the R group, was found to be 100%. Therefore, it was found that a pharmaceutical efficacy of infliximab against rheumatoid arthritis can be predicted by employing as an index an amount of ADAMTS5 before administration of infliximab.

--Comparison of GR group with NGR group--

The 30 cases with rheumatoid arthritis, who had been treated with infliximab, were categorized into the GR group (good response) and the NGR group (no response + moderate response). The expression level of ADAMTS5 mRNA before administration of infliximab was compared between the GR group and the NGR group by a real-time PCR method using a standard curve constructed with the whole cDNA from healthy human as a template DNA control. As a result, the ADAMTS5 mRNA expression level in the GR group was significantly lower than that in the NGR group (see Table 4).

Table 4

| ADAMTS5/$\beta$−actin(x1/25) | | |
|---|---|---|
| GR group ( n = 16 ) | 0.453 ± 0.227 | ⎤ |
| NGR group ( n = 14 ) | 0.682 ± 0.086 | ⎦ p = 0.0231 |

Also, as shown in Table 5, the cases whose ADAMTS5 mRNA expression level was lower than 0.6 × 1/25 were classified into the low ADAMTS5 group (Low), while the cases whose ADAMTS5 mRNA expression level was equal to or higher than 0.6 × 1/25 were classified into the high ADAMTS5 group (High).

Table 5

| ADAMTS5 | GR | NGR | Total |
|---|---|---|---|
| Low | 12 | 4 | 16 |
| High | 4 | 10 | 14 |
| Total | 16 | 14 | 30 |
| $\chi^2$ p = 0.0110 | | | |

Based on the classification in Table 5, percentages of sensitivity, specificity, positive predictive value, and negative predictive value were calculated according to the following equations. The results are shown in Table 6.

$$\text{Sensitivity (\%)} = E/(E + G) \times 100$$

$$\text{Specificity (\%)} = H/(H + F) \times 100$$

$$\text{Positive predictive value (PPV) (\%)} = E/(F + E) \times 100$$

$$\text{Negative predictive value (NPV) (\%)} = H/(H + G) \times 100$$

where E to G means as follows:

E: Number of cases classified into the low ADAMTS5 group and GR group
F: Number of cases classified into the low ADAMTS5 group and NGR group
G: Number of cases classified into the high ADAMTS5 group and GR group
H: Number of cases classified into the high ADAMTS5 group and NGR group

Table 6

| Sensitivity | 75.0% |
|---|---|
| Specificity | 71.4% |
| PPV | 75.0% |
| NPV | 71.4% |

As is clear from Table 6, the positive predictive value (PPV), which predicts that the cases classified into the low ADAMTS5 group would be classified into the GR group, was found to be high; i.e., 75.0%. Therefore, it was found that a pharmaceutical efficacy of infliximab against rheumatoid arthritis can be predicted by employing as an index an amount of ADAMTS5 before administration of infliximab.

--Comparison of Re group with Nre group --

The 30 cases with rheumatoid arthritis who were treated with infliximab were categorized into the Re group (remission) and the Nre group (non-remission). The expression level of ADAMTS5 mRNA before administration of infliximab was compared between the Nre group and the Re group by a real-time PCR method using a standard curve constructed with the whole cDNA from healthy human as a template DNA control. As a result, the ADAMTS5 mRNA expression level in the Re group was significantly lower than that in the Nre group (see Table 7).

Table 7

| $ADAMTS5/\beta-actin(x1/25)$ | | |
|---|---|---|
| Re group ( n = 14 ) | $0.434 \pm 0.231$ | $p = 0.0189$ |
| Nre group ( n = 16 ) | $0.670 \pm 0.078$ | |

Also, as shown in Table 8, the cases whose ADAMTS5 mRNA expression level was lower than $0.6 \times 1/25$ were classified into the low ADAMTS5 group (Low), while the cases whose ADAMTS5 mRNA expression level was equal to or higher than $0.6 \times 1/25$ were classified into the high ADAMTS5 group (High).

Table 8

| ADAMTS5 | remission | non-remission | Total |
|---|---|---|---|
| Low | 11 | 5 | 16 |
| High | 3 | 11 | 14 |
| Total | 14 | 16 | 30 |
| $\chi^2$ p = 0.0095 | | | |

Based on the classification in Table 8, percentages of sensitivity, specificity, positive predictive value, and negative predictive value were calculated according to the following equations. The results are shown in Table 9.

$$\text{Sensitivity (\%)} = I/(I + K) \times 100$$

$$\text{Specificity (\%)} = L/(L + J) \times 100$$

$$\text{Positive predictive value (PPV) (\%)} = I/(J + I) \times 100$$

$$\text{Negative predictive value (NPV) (\%)} = L/(L + K) \times 100$$

where I to L means as follows:

I: Number of cases classified into the low ADAMTS5 group and the Re group
J: Number of cases classified into the low ADAMTS5 group and the Nre group
K: Number of cases classified into the high ADAMTS5 group and the Re group
L: Number of cases classified into the high ADAMTS5 group and the Nre group

Table 9

| Sensitivity | 78.6% |
|---|---|
| Specificity | 68.8% |
| PPV | 68.8% |
| NPV | 78.6% |

As is clear from Table 9, the positive predictive value (PPV), which predicts that the cases classified into the low ADAMTS5 group would enter remission (classified into the Re group, was found to be high; i.e., 68.8%. Also, the negative predictive value (NPV), which predicts that the cases classified into the high ADAMTS5 group would not enter remission, was found to be high; i.e., 78.6%. Therefore, it was found that whether or not cases enter remission can be predicted by employing as an index an amount of ADAMTS5 before administration of infliximab.

--Prediction of DAS28 after administration of infliximab--

Fig. 1 shows correlation between the ADAMTS5 m RNA expression level of peripheral blood before administration of infliximab and the change of DAS28 at the end of 14 weeks of infliximab administration with respect to DAS28 before administration of infliximab (DAS% reduction).
As shown in Fig. 1, the ADAMTS5 m RNA expression level before administration of infliximab was found to be negatively correlated (r = -0.6) with the change of DAS28 at the end of 14 weeks of infliximab administration with respect to DAS28 before administration of infliximab (DAS% reduction).
That is, these results suggest that the lower an amount of ADAMTS5 contained in a sample derived from a subject before administration of infliximab, the more efficient an anti-TNF$\alpha$ antibody drug against rheumatoid arthritis. Thus, DAS28 after administration of an anti-TNF$\alpha$ antibody drug can be predicted by employing as an index an amount of ADAMTS5 contained in a sample derived from a subject before administration of infliximab.

<Comparison with previously reported case 1>

-Comparison with the report by Luquerre et al.-

The method of the present invention was compared with that of the report by Luquerre et al. (Laqurre T, et al. Arthritis Res Ther 2006; 8: R105). The report by Luquerre et al. predicted an efficacy of infliximab by employing a real-time PCR method and an algorithm.
Luquerre et al. compared DAS28 before infliximab administration with DAS28 at the end of 14 weeks after infliximab administration. They defined a case as an efficient case if DAS28 reduction ($\Delta$DAS) < 1.2 was observed. In this case, the positive predictive value (PPV) was 75% (20 transcripts) and 100% (8 transcripts).
On the other hand, the positive predictive value in Example 1 was 93.8% when calculated according to the same definition as in the report by Luquerre et al.
The present invention is superior to the report by Luquerre et al. in that the present invention uses a prospective study while Luquerre et al. use a retrospective study.

Table 10

| | | Present invention (Ex. 1) | Report by Luquerre et al. |
|---|---|---|---|
| Method of analysis | | Real-time PCR | Real-time PCR + algorithm |
| Sample used for predicting efficacy | | Blood before INF administration (0w) | Blood before INF administration (0w) |
| Type of study | | Prospective study | Retrospective study |
| Result of predicting efficacy (at 14w) | Definition | Efficient if $\Delta$DAS < 1.2 | Efficient if $\Delta$DAS < 1.2 |
| | Result | PPV = 93.8% | PPV = 75% (20 transcripts) PPV = 100% (8 transcripts) |

<Comparison with previously reported case 2>

-Comparison with the report by Trocme et al.-

The method of the present invention was compared with that of the report by Trocme et al. (Trocme C, et al. Ann Rheum Dis 2008. Epub ahead of print). The report by Trocme et al. predicted an efficacy of infliximab by employing a real-time PCR method and an algorithm.

Trocme et al. defined as an efficient case an ACR70 case in which 70% improvement was observed according to the ACR criteria, and defined as an inefficient case an under-ACR20 case where ACR20 means that 20% improvement was observed according to the ACR criteria. The "ACR criteria" was established by American College of Rheumatology in order to score degrees of improvement in rheumatoid arthritis symptoms and is described in Felson DT, et al. Arthritis Rheum 36: 729-740, 1993. The sensitivity and specificity based on the above definition were respectively 97.1% and 97.5%.

On the other hand, the sensitivity and specificity in Example 1 were respectively 75% and 100%, when calculated according to a definition almost the same as in Trocme et al. (good response group according to DAS28 was defined as efficient and no response group as inefficient).

The present invention is superior to the efficacy predicting study described in the report by Trocme et al. in that the present invention uses a prospective study while Trocme et al. use a retrospective study.

Table 11

| | | Present invention (Example 1) | Report by Trocme et al. |
|---|---|---|---|
| Method of analysis | | Real-time PCR | Real-time PCR + algorithm |
| Sample used for predicting efficacy | | Blood before INF administration (0w) | Blood before INF administration (0w) |
| Type of study | | Prospective study | Retrospective study |
| Result of predicting efficacy (at 14w) | Definition | good response: efficient no response: inefficient according to DAS28 | ACR70: efficient Under-ACR20: inefficient |
| | Result | Sensitivity = 75% Specificity = 100% | Sensitivity = 97.1% Specificity = 97.5% |

<Comparison with previously reported case 3>

-Comparison with RNA check by DNA Chip Research Inc.-

The method of the present invention was compared with the RNA check by DNA Chip Research Inc. The RNA check predicted an efficacy of infliximab by employing a microarray and an algorithm with a prospective study.

This kit defined an ACR50 case as an efficient case in which the positive predictive value (PPV) was 46.6% and the negative predictive value (NPV) was 44.4%.

On the other hand, the positive predictive value and the negative predictive value in Example 1 were respectively 75% and 71.4% when calculated according to a definition almost the same as the kit's definition (good response group according to DAS28 was defined as efficient).

Therefore, the percentage of efficacy prediction in the present invention is superior to that of the RNA check of DNA Chip Research Inc., although both use a prospective study.

Table 12

| | Present invention (Example 1) | RNA check (DNA Chip Research Inc.) |
|---|---|---|
| Method of analysis | Real-time PCR | Microarray + Algorithm |
| Sample used for predicting efficacy | Blood before INF administration (0w) | Blood before INF administration (0w) |

(continued)

|  | | Present invention (Example 1) | RNA check (DNA Chip Research Inc.) |
|---|---|---|---|
| Type of study | | Prospective study | Prospective study |
| Result of predicting efficacy (at 14w) | Definition | good response according to DAS28: efficient | ACR50: efficient |
| | Result | PPV = 75%<br>NPV = 71.4% | PPV = 46.6%<br>NPV = 44.4% |

Industrial Applicability

The method of the present invention for predicting a pharmaceutical efficacy of an anti-TNFα antibody drug against rheumatoid arthritis can predict an efficacy of an anti-TNFα antibody drug against rheumatoid arthritis with high reliability and in a simple manner as well as can predict activity of rheumatoid arthritis after administration of the anti-TNFα antibody drug by employing as an index an amount of ADAMTS5 at a time point before administration of the anti-TNFα antibody drug, resulting in that the anti-TNFα antibody drug can be administered to cases on which it assuredly takes effects to prevent the occurrence of unnecessary adverse side effects. Thus, the method of the present method can be suitably used for medical diagnosis and treatments.

The apparatus of the present invention for predicting a pharmaceutical efficacy of an anti-TNFα antibody drug against rheumatoid arthritis can predict an efficacy of an anti-TNFα antibody drug against rheumatoid arthritis with high reliability and in a simple manner as well as can predict activity of rheumatoid arthritis after administration of the anti-TNFα antibody drug by employing as an index an amount of ADAMTS5 at a time point before administration of the anti-TNFα antibody drug, resulting in that the anti-TNFα antibody drug can be administered to cases on which it assuredly takes effects to prevent the occurrence of unnecessary adverse side effects. Thus, the apparatus of the present method can be suitably used for medical diagnosis and treatments.

**Claims**

1. A method for predicting a pharmaceutical efficacy of an anti-TNFα antibody drug against rheumatoid arthritis, the method comprising:

   measuring an amount of ADAMTS5 contained in a sample derived from a subject, and
   determining that the anti-TNFα antibody drug is efficient against rheumatoid arthritis if the amount of ADAMTS5 is less than a prescribed value,
   wherein the amount of ADAMTS5 contained in the sample derived from the subject is an expression level of ADAMTS5 mRNA, and
   wherein the prescribed value is 0.6 x 1/25 as the expression level of ADAMTS5 mRNA measured using β-actin as an endogenous control (ADAMTS5/β-actin).

2. The method according to claim 1, wherein the anti-TNFα antibody drug is Infliximab (INF).

3. The method according to claim 1 or 2, wherein the expression level of ADAMTS5 mRNA is measured by a real-time PCR method.

4. An apparatus for predicting a pharmaceutical efficacy of an anti-TNFα antibody drug against rheumatoid arthritis, the apparatus comprising:

   a unit configured to measure an amount of ADAMTS5 contained in a sample derived from a subject, by measuring an expression level of ADAMTS5 mRNA and
   an electronic calculator configured to determine that the anti-TNFα antibody drug is efficient against rheumatoid arthritis if the amount of ADAMTS5 is less than a prescribed value,
   wherein the prescribed value is 0.6 x 1/25 as the expression level of ADAMTS5 mRNA measured using β-actin as an endogenous control (ADAMTS5/β-actin).

**5.** The apparatus according to claim 4, wherein the anti-TNFα antibody drug is Infliximab (INF).

**6.** The apparatus according to claim 4 or 5, wherein the expression level of ADAMTS5 mRNA is measured by a real-time PCR method.

**Patentansprüche**

**1.** Verfahren zur Vorhersage einer pharmazeutischen Wirksamkeit eines Anti-TNF-α-Antikörperwirkstoffs gegen rheumatoide Arthritis, wobei das Verfahren Folgendes umfasst:

Messen einer Menge an ADAMTS5, die in einer von einem Probanden stammenden Probe enthalten ist, und Bestimmen, dass der Anti-TNF-α-Antikörperwirkstoff wirksam gegen rheumatoide Arthritis ist, wenn die Menge an ADAMTS5 geringer ist als ein vorgeschriebener Wert,
wobei die Menge an ADAMTS5, die in der von dem Probanden stammenden Probe enthalten ist, ein Expressionsgrad von ADAMTS5-mRNA ist, und
wobei der vorgeschriebene Wert 0,6 x 1/25 beträgt, als Expressionsgrad von ADAMTS5-mRNA gemessen mit β-Actin als endogene Kontrolle (ADAMTS5/β-Actin).

**2.** Das Verfahren nach Anspruch 1, wobei der Anti-TNF-α-Antikörperwirkstoff Infliximab (INF) ist.

**3.** Das Verfahren nach Anspruch 1 oder 2, wobei der Expressionsgrad von ADAMTS5-mRNA mittels eines Real-Time-PCR-Verfahrens gemessen wird.

**4.** Eine Vorrichtung zur Vorhersage einer pharmazeutischen Wirksamkeit eines anti-TNF-α-Antikörperwirkstoffs gegen rheumatoide Arthritis, wobei die Vorrichtung Folgendes umfasst:

eine Einheit, die zur Messung einer Menge an ADAMTS5 in einer von einem Probanden stammenden Probe durch Messung eines Expressionsgrads an ADAMTS5-mRNA ausgelegt ist, und
ein elektronischer Rechner, der zur Bestimmung, dass der Anti-TNF-α-Antikörperwirkstoff wirksam gegen rheumatoide Arthritis ist, wenn die Menge an ADAMTS5 geringer ist als ein vorgeschriebener Wert, ausgelegt ist, wobei der vorgeschriebene Wert 0,6 x 1/25 beträgt, als Expressionsgrad von ADAMTS5-mRNA gemessen mit β-Actin als endogene Kontrolle (ADAMTS5/β-Actin).

**5.** Die Vorrichtung nach Anspruch 4, wobei der Anti-TNF-α-Antikörperwirkstoff Infliximab (INF) ist.

**6.** Die Vorrichtung nach Anspruch 4 oder 5, wobei der Expressionsgrad von ADAMTS5-mRNA mittels eines Real-Time-PCR-Verfahrens gemessen wird.

**Revendications**

**1.** Un procédé pour prédire une efficacité pharmaceutique d'un médicament à base d'anticorps anti-TNFα sur la polyarthrite rhumatoïde, le procédé comprenant :

mesurer une teneur de ADAMTS5 contenue dans un échantillon dérivé d'un sujet, et
déterminer si le médicament à base d'anticorps anti-TNFα est efficace contre la polyarthrite rhumatoïde si la teneur de ADAMTS5 est inférieure à une valeur prescrite,
dans lequel la teneur de ADAMTS5 contenue dans l'échantillon dérivé du sujet est un niveau d'expression de ADAMTS5 ARNm, et
dans lequel la valeur prescrite est 0,6 x 1/25 en tant que le niveau d'expression de ADAMTS5 ARNm mesuré en utilisant β-actine en tant qu'un contrôle endogène (ADAMTS5/β-actine).

**2.** Le procédé selon la revendication 1, dans lequel le médicament à base d'anticorps anti-TNFα est Infliximab (INF).

**3.** Le procédé selon la revendication 1 ou la revendication 2, dans lequel le niveau d'expression de ADAMTS5 ARNm est mesuré par une méthode reposant sur l'amplification en chaîne par polymérase (PCR) en temps réel.

4. Un appareil pour prédire une efficacité pharmaceutique d'un médicament à base d'anticorps anti-TNF$\alpha$ sur la polyarthrite rhumatoïde, l'appareil comprenant :

    une unité configurée pour mesurer une teneur de ADAMTS5 contenue dans un échantillon dérivé d'un sujet, en mesurant un niveau d'expression de ADAMTS5 ARNm et,
    un calculateur électronique configuré pour déterminer que le médicament à base d'anticorps anti-TNF$\alpha$ est efficace contre la polyarthrite rhumatoïde si la teneur de ADAMTS5 est inférieure à une valeur prescrite,
    dans lequel la valeur prescrite est 0,6 x 1/25 en tant que le niveau d'expression de ADAMTS5 ARNm mesuré en utilisant $\beta$-actine en tant qu'un contrôle endogène (ADAMTS5/ $\beta$-actine).

5. L'appareil selon la revendication 4, dans lequel le médicament à base d'anticorps anti-TNF$\alpha$ est Infliximab (INF).

6. L'appareil selon la revendication 4 ou la revendication 5, dans lequel le niveau d'expression de ADAMTS5 ARNm est mesuré par une méthode de PCR en temps réel.

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006092530 A

**Non-patent literature cited in the description**

- **LAQUERRE T ; GAUTHIER-JAUNEAU A ; BANSARD C ; DERAMBURE C ; HIRON M ; VITTECOQ1 O ; DAVEAU M ; MEJJAD O ; DARAGON A ; TRON F.** *Arthritis Res Ther,* 2006, vol. 8, R105
- **TROCME C ; MAROTTE H ; BAILLET A ; PALLOT-PRADES B ; GARIN J ; GRANGE L ; MIOSSEC P ; TEBIB J ; BERGER F ; NISSEN MJ.** *Ann Rheum Dis,* 2008
- **LAQUERRE T. et al.** Autoantibodies, metalloproteinases and bone markers in rheumatoid arthritis patients are unable to predict their responses to infliximab. *Rheumatology,* 2007, vol. 46, 446-453
- **FRANSEN J et al.** *Clin Exp Rheumatol,* 2005, vol. 23 (39), S93-S99
- Division of Rheumatology, Department of Internal Medicine. Saitama Medical Center. Saitama Medical University, October 2007
- **LAQURRE T et al.** *Arthritis Res Ther,* 2006, vol. 8, R105
- **TROCME C et al.** *Ann Rheum Dis,* 2008
- **FELSON DT et al.** *Arthritis Rheum,* 1993, vol. 36, 729-740